# EUROPEAN PATENT SPECIFICATION

(11) **EP 1 602 650 B1**
(45) Date of publication and mention of the grant of the patent: **24.10.2007**
(21) Application number: 05460013.5
(22) Date of filing: 27.05.2005
(51) Int. Cl.: C07D 281/16

(54) **Method for the preparation of quetiapine by reductive N-alkylation of 11-Piperazinodibenzo(B,F) (1,4)Thioazepin with an aldehyde**
Verfahren zur Hersterllung von Quetiapin durch reduktive N-Alkylierung von 11-Piperazinodibenzo(B,F) (1,4)Thioazepin mit einem Aldehyd
Procédé pour la préparation de quetiapine par une N-alkylation reductive de 11-Piperazinodibenzo(B,F) (1,4) Thioazepine avec un aldehyde

(30) Priority: 28.05.2004 PL 36824504
(43) Date of publication of application: 07.12.2005
(73) Proprietor: Celon Pharma Sp. z o.o., 05-092 Lomianki (PL)
(72) Inventor: Michalski, Krzysztof, 05-120 Legionow (PL); Wieczorek, Maciej, 05-092 Lomianki (PL)
(74) Representative: Witek, Rafal

(56) References cited:
- EP-A- 0 282 236
- DATABASE BEILSTEIN 2003, XP002342429 Database accession no. Reaction ID: 9678623 & CAPUANO ET AL: AUSTRALIAN JOURNAL OF CHEMISTRY, vol. 56, no. 9, 2003, pages 875-886,

## Description

The invention relates to new method of quetiapine synthesis, particularly ways of quetiapine synthesis using as reagents: piperazine derivatives of dibenzothioazepine (11-piperazindibenzo[b,f][1,4]thioazepine) and (2-hydroxyethoxy)acetaldehyde or its analogs, which has general formula III.
Quetiapine in the form of hemifumarate is known medicine acting on central nerve match and belongs in chemical respect to benzothioazepine derivatives (chemical name: 2-[2-(4-dibenzo[b,f][1,4]thioazepin-11-yl-1-piperazinyl)ethoxy]etanole). Mechanism of therapeutic action of quetiapine, just as other antipsychotic medicine (neuroleptics) applicable in treatment of schizophrenia, is average of antagonism for dopamine D2 and serotonin 5HT2 receptors.

According to European patent description EP 240228 quetiapine is received in reaction of proper iminochloride (11-chlorodibenzo[b,f][1,4]thioazepine) with 1-(2-hydroxyethoxy)ethylpiperazine. After purification by column chromatography oily substance is received with yield 77.7%. Employment of this process is tied in industrial scale with necessity arduous, lasting clean-up of products behind assistance of chromatographic columns and necessity of behave of proper environment conditions from the point of view of instability and hydrolysis capability in presence of wet from air earlier mentioned iminochloride.

According to European patent description No. 282236 piperazine derivative of earlier mentioned iminochloride (11-piperazindibenzo[b,f][1,4]thioazepin) is reacting with 2-haloethoxyethanol producing quetiapine with yield 78%. Employment of this method is tied with capability of revolt of dialkilation products mentioned earlier piperazine derivative, which is equal with necessity of sweeping of conduct of reaction conditions (strict control of temperatutre, reaction time, speed of alkylation factor addition). Separation of dialkilating by-products from monoalkilating products suggests big difficulty in industrial scale.

In patent application No. WO 01/55125 are presented alternative methods of quetiapine syntheses based on alkilation reakction of N-substituted haloalkylo derivatives of 11-piperazindibenzo[b,f][1,4]thioazepine by 1,2-dihydroxyethane (Wiliamson reaction), cyclization of halo- or hydroxyethylpiperazine derivatives in presence of deprotonation agent (base) and next reaction earlier created compound with 1,2-dihydroxyethane and N-alkilation of 1-(2-hydroxyethylo)piperazin by iminochloride 11-chlorodibenzo[b,f][1,4]thioazepine in presence of deprotonation agent (base) and chlorination agents (POCl₃, PCl₅) and next reaction earlier created compound with 1,2-dihydroxyethane.

Conducting of highly mentioned process is enough oppressive from the point of view of employment of metallic sodium in excess 1,5 - 1,7 mol for 1 mol dibenzothioazepine derivative (necessity of affirmation of without water environment), big excess of 1,2-dihydroxyethane (20-30 mol for 1 mol dibenzothioazepine derivative), high reaction temperature (50-150°C), very long reaction time (5-15 h) and using of very active and dangerous chlorination agents (POCl₃, PCl₅). All these factors effect economics negatively and facility of process of quetiapine synthesis.

The aim of this invention was new way of quetiapine synthesis, in which capability of pronouncement by-products is minimal, limits dangerous chlorination agents and metallic sodium and simplifies procedures of reactions conductions (reaction time, temperature, conditions without water, inert gas).

We have processed new way of quetiapine synthesis, in which as starting compounds are used piperazine derivative of dibenzothioazepine (11-piperazindibenzo[b,f][1,4]thioazepine) and aldehyde of formula III. This manner allows avoiding described above difficultis. 11-piperazindibenzo[b,f][1,4]thioazepin was described in European patent description No. 240228 and manufactured easily by N-monoalkilation of piperazin with known iminochloride 11-chlordibenz[b,f][1,4]thioazepine.

The new way of quetiapine (formula I) synthesis, according to invention relies on it, that 11-piperazindibenz[b,f][1,4]thioazepine (or its salts) of formula II is N-alkilated in reductive conditions by aldehyde of formula III.

In this manner according to invention 11-piperazindibenz[b,f][1,4]thioazepine is reductive alkilated by aldehyde III in the presence of metal borohydride (I or II group of periodic system) in water conditions in temperature between -10 do +20°C (due to that is gotten high yield and small amount of by-products).

Advantageously it applies borohydride of alkali metal, especially sodium borohydride.

Advantageously temperature of reaction is between 0 do 5°C. Reductive alkilation of 2° amines derivatives usually is carried out in methanol or ethanol in the presence of gas hydrogen under pressure and niclum of Ranney as a catalyst.
In the presented invention to reactor containing 11-piperazindibenz[b,f][1,4]thioazepine and corresponding aldehyde (formula III) solved in mixture of acetic acid and sodium acetate is added slowly corresponding borohydride. After reaction the mixture is neutralized by sodium hydroxide, extracting by diethyl ether, dryid by magnesium sulfate and cristalized in the form of white cristals.

### Example I.

Synthesis of quetiapine by reductive alkilation of 11-piperazindibenz[b,f][1,4]thioazepine with (2-hydroxyethoxy)acetaldehyde in the presence of sodium borohydride.

The mixture of 11-piperazindibenz[b,f][1,4]thioazepine (1 g, 0,0034 mol), sodium acetate (0,8 g, 0,01 mol), glacial acetic acid (4 cm³), (2-hydroxyethoksy)acetaldehyde (0,35g, 0,0034 mol) and water (50 cm³) is cooled to 0°C. Next sodium borohydride was slowly (40 min) added (5 g, 0,13 mol) in temperature 0°C.

After this time the mixture was stirred for 1 h in temperature 10°C and 10% aq NaOH was added (to pH about 8-9). The mixture was extracted with diethyl ether (3x30 cm³), organic faze was dried by sodium sulfate. The product (1.17g) was obtain with purity about 98% (HPLC)

### Example II.

Synthesis of quetiapine by reductive alkilation of 11-piperazindibenz[b,f][1,4]thioazepine with (2-hydroxyethoxy)acetaldehyde in the presence of magnesium borohydride.

The mixture of 11-piperazindibenz[b,f][1,4]thioazepine (1 g, 0,0034 mol), sodium acetate (0,8 g, 0,01 mol), glacial acetic acid (4 cm³), (2-hydroxyethoksy)acetaldehyde (0,35g, 0,0034 mol) and water (50 cm³) is cooled to 0°C. Next sodium borohydride was slowly (40 min) added (7 g, 0,13 mol) in temperature 0°C.

After this time the mixture was stirred for 1 h in temperature 10°C and 10% aq NaOH was added (to pH about 8-9). The mixture was extracted with diethyl ether (3x30 cm³), organic faze was dried by sodium sulfate. The product (1.09g) was obtain with purity about 90% (HPLC)

### Example III.

Synthesis of quetiapine by reductive alkilation of 11-piperazindibenz[b,f][1,4]thioazepine with ester of (2-hydroxyethoxy)acetaldehyde and acetic acid in the presence of sodium borohydride.

The mixture of 11-piperazindibenz[b,f][1,4]thioazepine (1 g, 0,0034 mol), sodium acetate (0,8 g, 0,01 mol), glacial acetic acid (4 cm³), ester of (2-hydroxyethoksy)acetaldehyde and acetic acid (0,50g, 0,0034 mol) and water (50 cm³) is cooled to 0°C. Next sodium borohydride was slowly (40 min) added (5 g, 0,13 mol) in temperature 0°C.

After this time the mixture was stirred for 1 h in temperature 10°C and 10% aq NaOH was added (to pH about 8-9). The mixture was extracted with diethyl ether (3x30 cm³), organic faze was dried by sodium sulfate. The product (1.00g) was obtain with purity about 82% (HPLC)

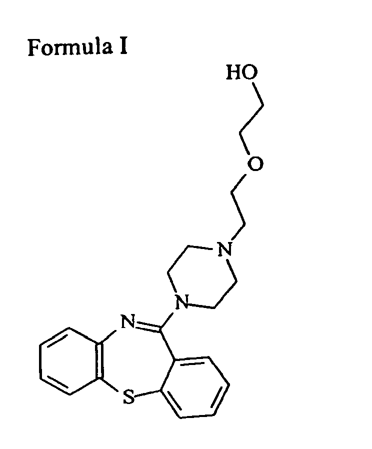

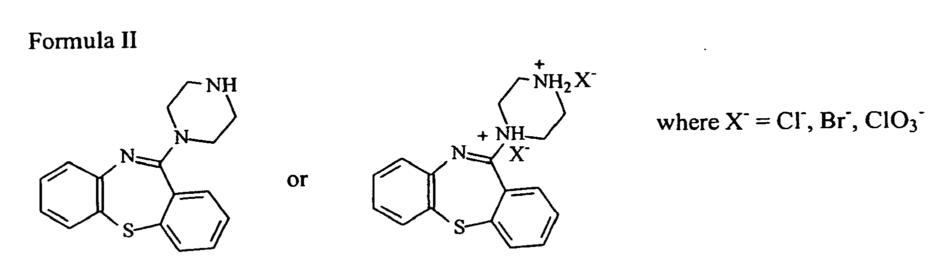

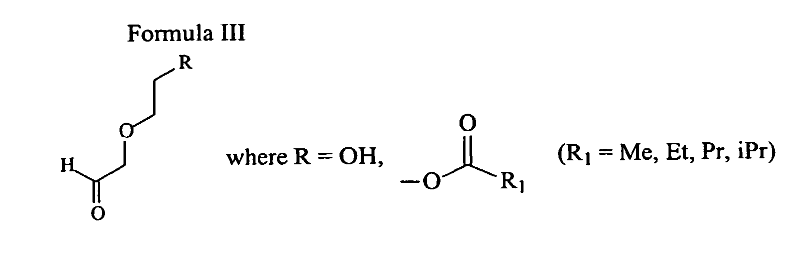

## Claims

1. Process for the manufacture of quetiapine **characterized in that** it comprises reductive N-alkilation of 11-piperazinodibenzo[b,f][1,4]thioazepin (or its salts) of formula II with aldehyde of formula III.

2. Process for the manufacture of quetiapine **characterized in that** it comprises reductive N-alkilation of 11-piperazinodibenzo[b,f][1,4]thloazepin (or its salts) of formula II with (2-hydroxyethoxy)acetaldehyd.

3. Process according to claims 1 and 2 **characterized in that** said reactions are carried out in the presence of metal (from I or II group of periodic table), particularly borohydride, favorably in the presence of sodium borohydride.

4. Process according to claims 1, 2 or 3 **characterized in that** it said reactions are caried out in the range of temperature between -10°C and 20°C, favourably in temperature between 0-5°C.

## Patentansprüche

1. Verfahren zur Herstellung von Quetiapin, **dadurch gekennzeichnet, daß** es die reduktive N-Alkylierung von 11-Piperazinodibenzo[b,f][1,4]thioazepin (oder seiner Salze) der Formel II mit einem Aldehyd der Formel III umfaßt.

2. Verfahren zur Herstellung von Quetiapin, **dadurch gekennzeichnet, daß** es die reduktive N-Alkylierung von 11-Piperazinodibenzo[b,f][1,4]thioazepin (oder seiner Salze) der Formel II mit (2-Hydroxyethoxy)acetaldehyd umfaßt.

3. Verfahren nach den Ansprüchen 1 und 2, **dadurch gekennzeichnet, daß** die Reaktionen in Gegenwart eines Metalls (aus der Gruppe I oder II des Periodensystems), insbesondere Tetrahydridoborats, günstigerweise in Gegenwart von Natriurntetrahydridoborat, durchgeführt werden.

4. Verfahren nach den Ansprüchen 1, 2 oder 3, **dadurch gekennzeichnet, daß** die Reaktionen in dem Temperaturbereich zwischen -10 und 20 °C, günstigerweise bei einer Temperatur zwischen 0 und 5 °C durchgeführt werden.

## Revendications

1. Processus de fabrication de quétiapine **caractérisé en ce qu'**il comprend la N-alkylation réductrice de 11-pipérazinodibenzo[b,f] [1,4]thioazépine (ou ses sels) de formule II avec un aldéhyde de formule III.

2. Processus de fabrication de quétiapine **caractérisé en ce qu'**il comprend la N-alkylation réductrice de 11-pipérazinodibenzo[b,f][1,4]thioazépine (ou ses sels) de formule II avec le (2-hydroxyéthoxy)acétaldéhyde.

3. Processus selon les revendications 1 et 2, **caractérisé en ce que** lesdites réactions sont réalisées en présence de métal (du groupe I ou II du tableau périodique), particulièrement de borohydrure, de manière favorable en présence de borohydrure de sodium.

4. Processus selon les revendications 1, 2 ou 3 **caractérisé en ce que** lesdites réactions sont réalisées dans la plage de températures comprise entre -10°C et 20°C, de manière favorable à une température comprise entre 0 et 5°C.
